# EUROPEAN PATENT APPLICATION

(11) **EP 3 570 033 A1**
(43) Date of publication of application: **20.11.2019**
(21) Application number: 18172392.5
(22) Date of filing: 15.05.2018
(51) Int. Cl.: G01N 33/574

(54) **EOSINOPHIL CATIONIC PROTEIN (EPC) AS A TUMOR MARKER FOR MALIGNANT TUMORS**

(71) Applicant: Friedrich-Alexander-Universität Erlangen-Nürnberg, 91054 Erlangen (DE)
(72) Inventor: Moreira, Alvaro, 91054 Erlangen (DE); Heinzerling, Lucie, 90419 Nürnberg (DE); Schuler, Gerold, 91080 Spardorf (DE)
(74) Representative: Winter, Brandl, Fürniss, Hübner, Röss, Kaiser, Polte - Partnerschaft mbB

(57) **Abstract**

The present invention relates to methods of prognosis of survival, of classifying a disease stage, and of selecting the mode of treatment of a patient diagnosed with malignant melanoma, a kit for determination of the level of eosinophil cationic protein (ECP) in a sample of a melanoma patient, and the use of this kit for methods of prognosis of survival, of classifying a disease stage and of selecting the mode of treatment of a patient diagnosed with malignant melanoma.

## Description

### Technical field

The present invention relates to a method of prognosis of survival of a patient diagnosed with malignant melanoma, a method of classifying a disease stage of a patient diagnosed with malignant melanoma, a method of selecting the mode of treatment of a patient diagnosed with malignant melanoma, wherein the aforementioned methods comprise determining the level of eosinophil cationic protein (ECP) in vitro in a sample of the patient, a kit for in vitro determination of the level of eosinophil cationic protein (ECP) in a sample of a patient, and the use of this kit for prognosis of survival of a patient diagnosed with malignant melanoma, for classifying a disease stage of a patient diagnosed with malignant melanoma, and for selecting the mode of treatment of a patient diagnosed with malignant melanoma.

### Background of the Invention

Established prognostic markers in melanoma - besides TNM stage - include LDH (lactate dehydrogenase) and performance status, while the tumor markers S100 B protein and protein melanoma-inhibitory-activity (MIA) are mostly used to detect progression of disease but do not correlate directly with prognosis.

Several studies have shown that eosinophil levels are linked with prognosis in different tumor entities. Increased frequencies of eosinophils were described to predict a better outcome in primary small cell oesophageal carcinoma and gastrointestinal, colorectal, breast and prostate cancer.

However, patients with eosinophilia (defined as at least 5% eosinophils in peripheral blood) show a worse prognosis in other tumor entities such as Hodgkin's lymphoma, oral squamous cell carcinoma or cervical carcinoma. Due to these inconsistent findings the role of eosinophils in tumor control is still not fully understood.

Eosinophil count has already been shown to be a predictive biomarker for therapy with immune checkpoint inhibitors in melanoma. Baseline frequencies as well as an increase of the number of eosinophils between the first and the second infusion of the anti-CTLA-4 antibody ipilimumab correlate with a better overall survival (OS) (Jacquelot N, Pitt JM, Enot DP, Roberti MP, Duong CPM, Rusakiewicz S, et al. Immune biomarkers for prognosis and prediction of responses to immune checkpoint blockade in cutaneous melanoma. Oncoimmunology. 2017;6).

Regarding therapy with anti-PD-1 antibodies, eosinophil count at baseline also correlated with OS of melanoma patients. Additionally, recent studies by the inventors revealed the prognostic value of eosinophils in melanoma patients (Moreira A, Leisgang W, Schuler G, Heinzerling L. Eosinophilic count as a biomarker for prognosis of melanoma patients and its importance in the response to immunotherapy. Immunotherapy [Internet]. 2017;9:115-21. Available from: http://www.futuremedicine.com/doi/10.2217/imt-2016-0138).

A prolonged survival was demonstrated in both cohorts of melanoma patients with eosinophilia, immunotherapy-naive and in patients receiving immunotherapy. However, in most cases patients only developed eosinophilia during the course of metastatic disease, thus eosinophil count at initial diagnosis of metastatic disease did not reliably predict survival.

Murine studies indicate that eosinophils are involved in CD8⁺ T cell-mediated tumor rejection by producing chemoattractants, such as CCL5, CXCL9 and CXCL10. Furthermore, studies on cancer patients also suggest that eosinophilic granulocytes affect tumor cells directly through the secretion of cytotoxic proteins.

Eosinophil-derived neurotoxin (EDN), for example, is associated with intratumoral cell apoptosis, but the role of other eosinophilic cytotoxins, like eosinophil cationic protein (ECP), eosinophil peroxidase (EPO) or major basic protein (MBP) has not yet been established.

ECP serves as a ribonuclease and belongs to RNase A family 3. Its release can be induced by immunoglobulins (IgE, IgG), surface-bound complement as well as lipid mediators (lipopolysaccharides (LPS) or Lipid A). Though ECP's ribonucleolytic activity is low, its cell membrane binding mediates a multitude of further functions, like osmotic lysis, synthesis of reactive oxygen species, reversed membrane asymmetry, chromatin condensation as well as increased Caspase-3-like activity and, thus, cytotoxicity as shown in mammalian cell culture models (Navarro S, Aleu J, Jimenez M, Boix E, Cuchillo CM, Nogues M V. The cytotoxicity of eosinophil cationic protein/ribonuclease 3 on eukaryotic cell lines takes place through its aggregation on the cell membrane. Cell Mol Life Sci [Internet]. 2008 [cited 2017 Dec 27];65:324-37. Available from: http://www.ncbi.nlm.nih.gov/pubmed/18087674).

It was suggested that ECP might, aside from harming various microorganisms also have cytotoxic activity against cancer cells or promote tumor infiltration through muscle fiber corrosion, but its definite role in human cancer is yet to be investigated. Moreover, in vitro studies showed that ECP inhibits immune functions such as the production of immunoglobulins as well as T cell proliferation. However, the role of ECP *in vivo* has to be determined.

Lose and Frandsen, 1989: "Eosinophil cationic protein in urine in patients with urinary bladder tumors"; Urol Res. 1989;17(5):295-7 suggests measurement of eosinophil cationic protein (ECP) in patient urine for use in the diagnosis of bladder tumor, in particular for a prognosis of survival of a patient. According to this document, it appears that increased ECP levels are associated with tumor presence.

Regarding the need for prognosis of survival of patients in later stages of melanoma, such as in metastasized melanoma of stage IV, lactate dehydrogenase (LDH) is currently commonly used therefor (Weinstein et al., J Clin Aesthet Dermatol. 2014; 7(6):13-24).

In the current version of the TNM classification scheme recently published by the American Joint Committee of Cancer (AJCC), LDH is the only biomarker used in exact staging of stage IV patients suffering from metastasized melanoma (Gershenwald JE, Scolyer RA, Hess KR, Sondak VK, Long GV, Ross MI et al (2017): Melanoma staging: evidence-based changes in the American joint committee on cancer eighth edition cancer staging manual. Ca Cancer J Clin 67:472-492. https://doi.org/10.3322/caac.21409). This staging is relevant for stratification of patients for clinical studies as well as for establishing a prognosis of survival of patients.

However, LDH is commonly known as an unspecific marker, the level of which is being influenced by numerous factors. Use of LDH as a biomarker is currently being discussed among experts and in the literature (cf. "The Puzzling Prognostic Effect of LDH in Melanoma", Jeffrey S. Weber, MD, PhD, March 06, 2017; https://www.medscape.com/viewarticle/876529).

Thus, it appears problematic in view of the present knowledge on LDH to give determination of LDH levels such high relevance for a meaningful assessment of patients' stages and prognosed survival.

Therefore, novel markers are required for (clinical) staging of patients as well as for establishing a more meaningful prognosis of survival for patients.

Recent advances in the therapy of melanoma patients have led to a variety of effective new drugs which may be used for the treatment of melanoma patients. While immunotherapies such as anti-CTLA4 or anti-PD1 as well as a combination of the two show promising effects in patients suffering from metastasized melanoma and allow a prolonged effective use in patients, therapies with these drugs suffer from pronounced side effects such as inflammatory reactions which can cause irreversible damages and fatalities as well as a comparably later onset of therapeutic effects. Importantly, since anti-PD1 is effective in a subgroup of patients but better tolerated it would be of interest who responds to anti-PD1 alone to spare additional toxicity from the application of ipilimumab. Patients previewed to respond poorly could directly be treated with combination immunotherapy.

In contrast, the alternative class of drugs termed "targeted therapies" comprises inhibitors of the B-Raf and MEK kinases. These agents show an earlier onset of therapeutic effects, easier manageable side effects that do not cause sequelae but have been reported as having a comparatively reduced average duration of action.

Based on the different profiles of action of these classes of melanoma drugs, it is desired to establish additional criteria to determine and select a mode of treatment which is most appropriate and suitable for the patient to be treated.

Taken together, it is an object of the present invention to provide a method of assessing the status of a malignant tumor of a patient.

Further, it is an object of the present invention to provide a method for a more meaningful prognosis of survival of a patient diagnosed with melanoma.

It is another object of the present invention to provide a method for predicting the response to therapy of a patient diagnosed with a malignant tumor.

It is yet another object of the present invention to provide a method for classifying a (clinical/prognostic) stage of a patient diagnosed with melanoma.

It is another object of the present invention to provide a method for monitoring disease control in tumor patients.

Furthermore, it is an object of the present invention to provide a method of selecting an appropriate mode of treatment of a patient diagnosed with melanoma which is based on well-founded information.

Ultimately, it is another object of the present invention to provide a kit which allows point-of-care testing for the assessment of the status of a malignant tumor of a patient, , and the use of said kit for assessing the status of a malignant tumor, and prognosis of survival, predicting response to therapy, classifying a stage, selecting an appropriate mode of treatment, or monitoring disease control of a patient diagnosed with melanoma.

### Summary of the Invention

These objects have been solved by the aspects of the present invention as specified hereinafter.

According to the first aspect of the present invention, a method of assessing the status of a malignant tumor, preferably melanoma, of a patient is provided, the method comprising determining the level of expression or concentration of eosinophil activation proteins, preferably of eosinophil cationic protein (ECP), *in vitro* in a sample of the patient.

According to the second aspect of the present invention, eosinophil activation proteins, preferably eosinophil cationic protein (ECP), are provided for use as a biomarker in a method of assessing the status of a malignant tumor, preferably melanoma, wherein the use comprises determining the level of expression or concentration of eosinophil activation proteins, preferably of eosinophil cationic protein (ECP), wherein the determination is practised on the human or animal body.

According to a preferred embodiment of the first or second aspect of the present invention, the method of assessing the status of a malignant tumor is a method of prognosis of survival of a patient diagnosed with a malignant tumor.

According to another preferred embodiment of the first or second aspect of the present invention, the method of assessing the status of a malignant tumor is a method of predicting response to therapy of a patient diagnosed with a malignant tumor.

According to another preferred embodiment of the first or second aspect of the present invention, the method of assessing the status of a malignant tumor is a method of classifying a disease stage, preferably a prognostic disease stageof a patient diagnosed with a malignant tumor.

According to another preferred embodiment of the first or second aspect of the present invention, the method of assessing the status of a malignant tumor is a method of selecting the mode of treatment of a patient diagnosed with melanoma.

According to another preferred embodiment of the first or second aspect of the present invention, the method of assessing the status of a malignant tumor is a method of monitoring disease control in tumor patients.

In a preferred embodiment of the present invention, level of expression or concentration of eosinophil activation proteins, preferably of eosinophil cationic protein (ECP), is associated with survival or correlated to response to therapy.

In another preferred embodiment of the present invention, the level of the eosinophil activation proteins or mRNA coding for eosinophil activation proteins, preferably of eosinophil cationic protein (ECP) or mRNA coding for eosinophil cationic protein (ECP) is determined in a tumor tissue sample or in a body fluid, preferably in blood, serum, urine, or plasma of a patient, more preferably in the serum of a patient.

In yet another preferred embodiment of the present invention, the level of eosinophil activation proteins, preferably eosinophil cationic protein (ECP), associated with a negative status, preferably a negative prognosis of survival or a negative prediction for response to therapy, is above 10 ng/ml, 16 ng/ml, 20 ng/ml, 25 ng/ml, 40 ng/ml, or 60 ng/ml, preferably above 16 ng/ml or 10 ng/ml, more preferably above 10 ng/ml.

In another preferred embodiment of the present invention, the level of expression or concentration of eosinophil activation proteins is determined by a method indicative of ECP expression in the patient, preferably the level of expression or concentration of eosinophil activation proteins is determined by RNA analysis.

In another preferred embodiment of the present invention, the level of eosinophil activation proteins, preferably eosinophil cationic protein (ECP), associated with a positive status, preferably a positive prognosis of survival or a positive prediction for response to therapy, is below 60 ng/ml, 40 ng/ml, 25 ng/ml, 20 ng/ml, 16 ng/ml, or 10 ng/ml, preferably below 16 ng/ml or 10 ng/ml, more preferably below 10 ng/ml..

According to a preferred embodiment of the present invention, the patient status is determined in comparison to the general comparative patient population at the time of diagnosis of disease, preferably wherein a positive and/or a negative status is determined in comparison to the general comparative patient population at the time of diagnosis of disease.

According to a more preferred embodiment of the present invention, the time of diagnosis of metastatic disease is defined as within 0-6 months from the date of diagnosis of disease or before start of a treatment or during a treatment, preferably within 0-6 months from the date of diagnosis of stage IV melanoma, more preferably from time of eosinophilia.

According to a preferred embodiment of the present invention, the level of expression or concentration of eosinophil activation proteins, preferably eosinophil cationic protein (ECP), is determined by using a qualitative or semiquantitative or quantitative method selected from fluoroenzyme immunoassay, immunohistochemistry, Western blot, flow cytometry, cytokine bead array analysis or ELISA, preferably wherein the level of expression or concentration of eosinophil activation proteins, preferably eosinophil cationic protein (ECP), is determined by using ELISA.

According to the third aspect of the present invention, a kit is provided for *in vitro* determination of the level of eosinophil activation proteins or mRNA of eosinophil activation proteins, preferably eosinophil cationic protein (ECP) or ECP mRNA, in a sample of a patient.

According to a preferred embodiment of the third aspect of the present invention, the kit comprises reagents comprising one or more recognition molecules specifically binding to eosinophil activation proteins, preferably eosinophil cationic protein (ECP), wherein the reagents are suitable to detect eosinophil activation proteins, preferably eosinophil cationic protein (ECP) in a patient sample, more preferably wherein the reagents are suitable for quantification of the level of eosinophil activation proteins, preferably eosinophil cationic protein (ECP), in a patient sample.

According to a more preferred embodiment of the third aspect of the present invention, the patient is suffering from a malignant tumor, preferably melanoma, more preferably wherein the patient has been diagnosed with metastatic disease, even more preferably wherein the patient has been diagnosed within 0-6 months or before start of a treatment or during a treatment.

According to one preferred embodiment of the third aspect of the present invention, the patient sample is tumor tissue or a body fluid, more preferably blood, serum or plasma of a patient, particularly preferably serum of a patient.

According to the fourth aspect of the present invention, the use of the kit of the third aspect of the present invention is provided for assessing the status of a malignant tumor, preferably for prognosis of survival of a patient diagnosed with a malignant tumor, preferably melanoma, and/or for predicting response to therapy of a patient diagnosed with a malignant tumor, preferably melanoma, and/or for classifying a stage of a patient diagnosed with a malignant tumor, preferably melanoma, and/or for selecting the mode of treatment of a patient diagnosed with a malignant tumor, preferably melanoma, or for monitoring disease control of a patient diagnosed with a malignant tumor, preferably melanoma.

### Description of Figures

Figure 1 shows the overall survival of patients distinguished by ECP levels with a cut-off at 16 ng ECP / ml of sample.
Figure 2 shows the overall survival of patients distinguished by ECP levels with a cut-off at 10 ng ECP / ml of sample.
Figure 3 shows a comparison between pretreatment ECP and LDH levels at initial diagnosis of metastatic disease.

### Detailed Description of the Invention

The present inventors have studied the role of eosinophils in the progression of metastasized melanoma. In the course of their research, they have surprisingly recognised that while eosinophilia in melanoma patients may point to a prolonged survival, the correlation is reversed regarding the levels of ECP in patients' samples.

In the present invention, "ECP" or "eosinophil cationic protein" may be used interchangeably for the same protein. The amino acid sequence of ECP is commonly known and may preferably be accessed as the entry P12724 in the database UniProt/UniProtKB.

In the context of the present invention, processes marked to be carried out *"in vitro"* are preferably not to be carried out and/or practised on the human or animal body and *vice versa.* Also herein, methods or products for use *"in vivo"* are preferably to be carried out, practised or used on the human or animal body and *vice versa.*

Thus, the inventors for the first time found that decreased ECP levels in samples of patients serve to indicate a prolonged survival, while patients exhibiting increased levels of ECP in their samples may have reduced survival rates. The present inventors recognized that ECP represents an independent novel prognostic biomarker in patients with metastatic melanoma. This recognition allows to assess the status of a tumor and/or the status of a patient being diagnosed and/or suffering from said tumor in many different ways. Preferably, an assessment of such a status should be understood to include the prognosis of survival, prediction of response to therapy, classifiying a stage, selecting an appropriate mode of treatment, monitoring disease control, and others.

Patients with higher ECP levels at the time of diagnosis of metastatic disease have a shorter survival compared to patients with lower ECP serum levels.

Interestingly, ECP was associated with a worse outcome although it is secreted by eosinophils whose presence was previously found to be positively correlated with overall survival in melanoma. This reverse correlation with survival was observed irrespective of the kind of therapy the patients were receiving and irrespective of the presence of eosinophilia. ECP's reported cytotoxicity against cancer cells *in vitro* thus does not correspond to its presently recognized disadvantageous role in melanoma patients *in vivo.*

Since ECP is a granule cytotoxic protein of eosinophils, it could have been assumed that eosinophilia precedes high ECP serum levels. However, this does not appear to be the case. Only 15% of patients with elevated ECP show eosinophilia compared to 9% with low ECP (data not shown). In the course of metastatic disease 32% of patients with low ECP and 47% of patients with elevated ECP developed eosinophilia (data not shown). Thus, elevated levels of ECP are not directly associated with the presence of eosinophilia.

Furthermore, there was no correlation of ECP with LDH. Although the function of ECP in cancer progression or rejection is still not fully understood, its levels were associated with prognosis in patients with metastatic melanoma. So ECP seems to represent a novel independent prognostic biomarker in melanoma and perhaps other tumors as well. Thus, within the present invention, ECP may be used as a biomarker in different types of tumors. However, melanoma is preferred as the type of tumor. The measurement of this liquid biomarker in routine clinical practice would be easy and time efficient. The inventors are, therefore, currently validating ECP as a new biomarker.

This recognition allows for the first time an indication of expected survival which can be obtained by simple experimental procedures without the need for complex medical machinery. Based on the results obtained through determination of ECP levels, a more exact clinical staging can be carried out which will then guide selection of patients for clinical studies, and others. Last, based on the determined overall survival, the treating clinician and/or physician may select an appropriate treatment form for the patient in question.

Overall, ECP is a novel prognostic serum marker for the outcome of melanoma patients, which is independent of LDH and easy to perform in clinical practice. The negative prognostic value of high ECP level is unanticipated. Based on the results obtained with ECP, the present invention is based on the determination of the level of expression or concentration of eosinophil activation proteins, wherein ECP is preferred.

Thus, according to the present invention, a method of prognosis of survival of a patient diagnosed with malignant melanoma is provided, the method comprising determining the level of eosinophil cationic protein (ECP) *in vitro* in a sample of the patient.

The method of prognosis is preferably carried out to determine an expected median survival (e.g. in months) of patients suffering from melanoma.

Further, the present invention provides a method of classifying a stage of a patient diagnosed with melanoma, the method comprising determining the level of eosinophil cationic protein (ECP) *in vitro* in a sample of the patient. The determination of ECP levels *in vitro* in a patient sample can preferably be used to assign a patient to a specific clinical or prognostic stage. According to a preferred embodiment, the determination of ECP levels can be used to introduce new stages and/or classifications for a more exact designation or evaluation of melanoma patients.

Also, the present invention provides a method of selecting the mode of treatment of a patient diagnosed with melanoma, the method comprising determining the level of eosinophil cationic protein (ECP) *in vitro* in a sample of the patient. Further the present invention provides a method of predicting response to therapy of a patient diagnosed with a malignant tumor, preferably melanoma.

Treatment of melanoma patients is available by different medication schemes and different classes of substances. Since all of these different treatments have certain characteristics, advantages and disadvantages, a more exact prediction of response and overall survival will assist the treating clinician and/or physician with the selection of an appropriate treatment regime.

Based on the measurement of ECP in the patient's sample, median overall survival of the patient may be determined.

If the median overall survival is expected to be low (e.g. less than 24 months, less than 18 months, less than 12 months, less than 6 months) due to high levels of ECP determined in the patient's sample, it is preferred to select a mode of treatment with an early onset of therapeutic effect and a higher response rate while accepting higher toxicity. If median overall survival is expected to be low (e.g. less than 24 months, less than 18 months, less than 12 months, less than 6 months), it could also be appropriate to choose palliative treatment. Also, if median overall survival is expected to be low (e.g. less than 24 months, less than 18 months, less than 12 months, less than 6 months), it is preferred to change the selected treatment at an earlier point in time if no effects can be observed.

Based on the above considerations, it is preferred to treat the patient having high levels of ECP determined in the patient's sample with a targeted therapy, e.g. B-Raf and/or MEK kinases or combination immunotherapy. However, it is well known in the art that these therapies may not be applicable to any patient and their suitability for the individual patient must be determined before application as is commonly done in the art.

On the other hand, if the median overall survival is expected to be high (e.g. more than 6 months, more than 12 months, more than 18 months, more than 24 months) due to low levels of ECP determined in the patient's sample, it is preferred to select a mode of treatment with a comparably later onset of therapeutic effect, if said treatment promises other advantages. It is also preferred to select a mode of treatment of patients with a median overall survival expected to be high due to low levels of ECP determined in the patient's sample which allows a prolonged effective use of the treatment, and/or a treatment which is better tolerated.

Aspects such as comparably increased or pronounced side effects or late onset of a therapeutic effect may be given less relevance in view of the advantages of certain drugs as mentioned above. Also, the results of predicting a response to a certain therapy based on a determination of eosinophil activation proteins, preferably ECP, is preferably taken into account when selecting an appropriate mode of treatment.

Based on the above considerations, it may be preferred to treat a patient having low levels of ECP determined in the patient's sample with an immunotherapy, e.g. anti-CTLA4 or anti-PD1 antibodies or a combination of the two.

As already mentioned above, the present invention is based on the recognition that higher levels of ECP in the patient sample are associated with decreased mean survival and that lower levels of ECP in the patient sample are associated with increased mean survival.

According to a preferred embodiment of the present invention, the level of eosinophil cationic protein is determined in any sample of the patient including solid samples such as tumor tissue, or liquid samples, such as a body fluid, preferably a fluid of a human body, more preferably in human blood, plasma, serum, urine, feces, synovial fluid, interstitial fluid, lymph, saliva, spinal fluid and/or lacrimal fluid, particularly preferably in human blood, plasma or serum, most preferably in the serum of a patient. Preferably, RNA levels indicative for expression of ECP may be determined in tumor tissue, more preferably the level of mRNA coding for ECP.

Increased ECP levels in comparison to decreased ECP levels which are indicative of reduced or increased mean survival, respectively, may be different for each of the different types of patient samples. However, respective cut-off values for a meaningful interpretation of mean survival associated with ECP levels in a specific type of sample may be determined by the skilled person as part of their routine activities and based on their general knowledge and skill without requiring undue experimentation or inventive skill.

Preferably, a higher level of eosinophil cationic protein (ECP) in the patient sample which is associated with a specific status, preferably a more negative status, more preferably decreased mean survival is above 8 ng/ml, more preferably above 9 ng/ml, even more preferably above 10 ng/ml, even more preferably above 11 ng/ml, even more preferably above 12 ng/ml, even more preferably above 13 ng/ml, even more preferably above 14 ng/ml, even more preferably above 15 ng/ml, even more preferably above 16 ng/ml, even more preferably above 17 ng/ml, even more preferably above 18 ng/ml, even more preferably above 20 ng/ml, even more preferably above 25 ng/ml, even more preferably above 40 ng/ml, even more preferably above 60 ng/ml. A particularly preferred level of eosinophil cationic protein (ECP) in the patient sample which is associated with decreased mean survival is above 10 ng/ml, more preferably above 16 ng/ml.

Preferably, a lower level of eosinophil cationic protein (ECP) in the patient sample which is associated with a specific status, preferably a more positive status, more preferably increased mean survival is below 60 ng/ml, more preferably below 40 ng/ml, even more preferably below 25 ng/ml, even more preferably below 20 ng/ml, even more preferably below 18 ng/ml, more preferably below 17 ng/ml, even more preferably below 16 ng/ml, even more preferably below 15 ng/ml, even more preferably below 14 ng/ml, even more preferably below 13 ng/ml, even more preferably below 12 ng/ml, even more preferably below 11 ng/ml, even more preferably below 10 ng/ml, even more preferably below 9 ng/ml, even more preferably below 8 ng/ml. A particularly preferred level of eosinophil cationic protein (ECP) in the patient sample which is associated with increased mean survival is below 16 ng/ml, more preferably below 10 ng/ml.

A specific status in the context of the present invention may preferably mean a status which is different from the average status of the general comparative patient population. A more positive status in this context may preferably mean an increased mean survival and/or a better prediction of a response to a therapy and/or a classification to a stage associated with better prognosis and/or selection of a more promising mode of treatment and/or better results in the monitoring of disease control. A more negative status in this context may preferably mean a decreased mean survival and/or a worse prediction of a response to a therapy and/or a classification to a stage associated with worse prognosis and/or selection of a less promising mode of treatment and/or worse results in the monitoring of disease control.

Decreased and/or increased mean survival in the context of the present invention may preferably be determined in comparison to the mean survival of the general comparative patient population at the time of diagnosis of metastatic disease. Alternatively preferably the decreased mean survival of a patient subgroup having higher levels of ECP in their patient samples is determined in comparison to the patient subgroup having lower levels of ECP in their patient samples.

In a preferred embodiment of the present invention, the patient addressed herein is suffering from melanoma, more preferably the patient has been diagnosed with metastatic melanoma, even more preferably wherein the patient has been diagnosed with stage IV metastatic melanoma in the last 0-6 months. Preferably, patients are addressed which have been diagnosed with stage IV metastatic melanoma within the last 6 months. Thus, the time of diagnosis of metastatic disease is preferably defined herein as within 0-6 months from the date of stage IV diagnosis.

Preferably, the level of eosinophil cationic protein is determined in the sample by methods commonly known and used for the determination of protein levels in a sample. More preferably, the method used for quantification of the ECP levels in a sample is selected from the group including immunohistochemistry, Western blot, flow cytometry, or ELISA, wherein ELISA is most preferred.

Furthermore, the present invention provides a kit for *in vitro* determination of the level of eosinophil cationic protein (ECP) in a sample of a patient. Preferably, the kit comprises reagents comprising one or more recognition molecules specifically binding to eosinophil cationic protein (ECP), wherein the reagents are suitable to detect eosinophil cationic protein (ECP) in a patient sample, preferably wherein the reagents are suitable for quantification of the level of eosinophil cationic protein (ECP) in a patient sample.

According to one preferred embodiment, the kit comprises reagents suitable for quantification of the ECP levels in a sample by one of immunohistochemistry, Western blot, flow cytometry, or ELISA, wherein reagents for quantification of ECP levels by ELISA are most preferred.

Preferably, the kit of the present invention should be suitable for simple and straightforward use at the point of care and/or bedside use.

The present invention also provides the use of the kit of the present invention as disclosed above for assessing the status of a malignant tumor, predicting response to therapy, prognosis of survival of a patient diagnosed with melanoma, and/or for classifying a stage of a patient diagnosed with melanoma, and/or for selecting the mode of treatment of a patient diagnosed with melanoma, and/or for monitoring disease control of a patient diagnosed with melanoma.

As already explained above, the determination of ECP levels in a patient sample allows for subsequent prognosis of survival, classifying a stage of the disease and selecting a suitable mode of treatment for this patient. Thus, any more detailed description of the uses mentioned above which are present herein should be understood to also relate to the use of the kit provided by the present invention.

All embodiments of the present invention as described herein are deemed to be combinable in any combination, unless the skilled person considers such a combination to not make any technical sense.

### Examples

### Patients and clinical characteristics

In total, 56 patients with metastatic melanoma treated from January 2004 to September 2017 were included in this study and analyzed retrospectively. Variables that were analyzed include gender, age, tumor involvement, type of melanoma, systemic therapies, and overall survival.

The patient cohort incorporated patients independently of their therapy, including patients who had received chemotherapy, radiotherapy, surgery or immune checkpoint inhibitors. The patient characteristics are depicted in table 1.

**Table 1**

| **Variables** | | **All patients (n** = **56)** | |
|---|---|---|---|
| Age | Median (Range) | 61.5 years (31 - 90 years) | |
| Gender | | n | % |
| | Male | 36 | 64 |
| | Female | 20 | 36 |
| Type of melanoma | Cutaneous* | 32 | 57 |
| | Mucosal* | 7 | 13 |
| | Uveal | 4 | 7 |
| | MUP | 14 | 25 |
| LDH | > ULN | 38 | 68 |
| | < ULN | 18 | 32 |
| Brain metastases (M1d) | | 26 | 46 |
| OS | Median (Range) | 11.5 months (2-48 months) | |

The cohort included all histological types of melanoma (cutaneous melanoma, mucosal melanoma, uveal melanoma and melanoma of unknown primary). Blood sera routinely assessed for tumor markers were used for analysis of eosinophil cationic protein (ECP).

### Determination of ECP in serum

For determination of ECP blood sera stored at -20°C were thawed and measured by enzyme-linked immunosorbent assay ELISA (CSB-E11729h; Cusabio; Erlangen) with a detection range of 1.56-100 ng/ml according to manufacturer's protocol.

Sera from the time at initial diagnosis of metastatic melanoma were taken defining the initial diagnosis as 0-6 months from the date of stage IV diagnosis. Duplicates of each sample were measured. Serum levels of at least 16.0 ng/ml were defined as elevated, because healthy individuals have a 95% range from 2.3-15.9 ng/ml in the serum (29).

Additionally, a cut-off of 10.0 ng/ml was analyzed and correlated with survival.

### Determination of LDH and blood counts

Serum lactate dehydrogenase (LDH) and blood counts were routinely measured in our lab. LDH was analyzed by means of the LDH-ratio (actual value divided by the upper limit of normal). Eosinophilia was defined as at least 5% eosinophils in peripheral blood counts.

In 4 of 56 cases eosinophil counts were not analyzed at the time of serum assessment. In these patients the eosinophil counts closest to the serum assessment was taken i.e. a maximum of 4 months before or after.

### Statistical methods

Event-time distributions were estimated with the Kaplan-Meier method. Both Log-Rank (Mantel-Cox) test as well as Gehan-Breslow-Wilcoxon test were performed to determine the p-value. For contingency analyses Fisher's exact test was utilized. Graphing was created using GraphPad Prism.

### ECP is inversely correlated with survival

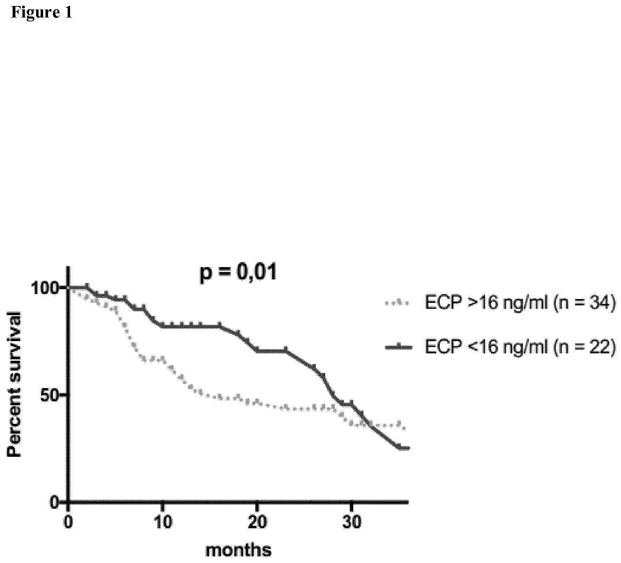

Patients with low ECP at initial diagnosis of metastatic disease had a longer survival in comparison with patients with high ECP. With a cut-off at 16.0 ng/ml serum ECP, the median OS for patients with ECP levels of at least 16.0 ng/ml (N = 34) was 16 months, compared with 28 months for patients with levels below this threshold (N = 22) (p = 0.01; Fig. 1).

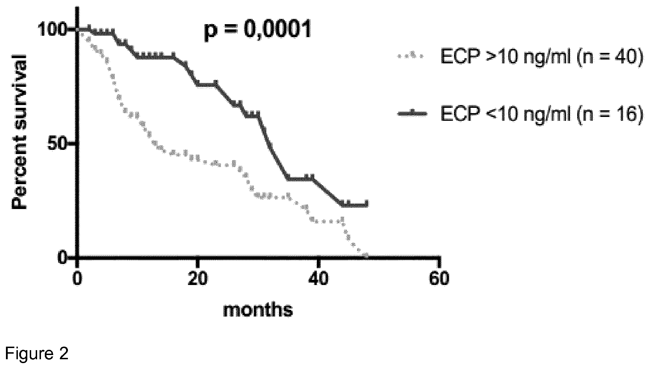

Dichotomizing at 10.0 ng/ml, patients with higher serum levels (N = 40) had a median OS of 13 months, compared with 32 months for patients below this threshold (N = 16) (p = 0.0001; Fig. 2). Immunotherapy-naive patients were analyzed and compared to patients who received immunotherapy and no difference was found (data not shown).

### ECP and eosinophilia

In order to investigate whether occurrence of eosinophilia (defined as at least 5% eosinophils in peripheral blood) was linked to presence of ECP, eosinophil counts in the course of the metastatic disease were analyzed and compared to ECP serum levels.

From the patients with decreased ECP serum levels (defined as <16.0 ng/ml, N = 22) at diagnosis of metastatic disease, only 9% (N = 2) had eosinophilia at that time (data not shown). Regarding the patients with increased ECP levels (N = 34), eosinophilia was also present in only 15% (N = 5) of the patients (data not shown).

Within the patients with initially increased ECP levels, 47% (16/34) had at some point during the course of their disease an eosinophilia, whereas 32% (7/22) of the patients with decreased ECP also experienced one. In 4 of these 7 patients, blood sera at time of occurrence of eosinophilia could be analyzed as well. Interestingly, 75% (3/4) of them still had decreased ECP levels when eosinophilia peak was reached (average ECP value = 6.1 ng/ml; data not shown).

Regarding development of eosinophilia after ECP assessment, no significant difference (p = 0.2824) could be found between patients with decreased ECP values and patients with increased ECP values.

### ECP is independent of LDH

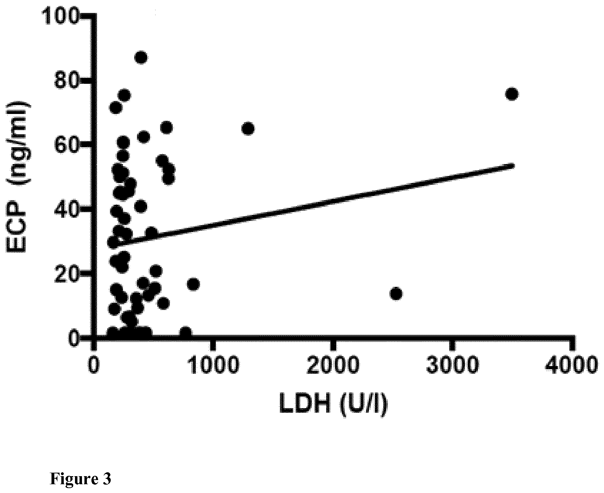

In order to investigate the role of ECP as an independent biomarker, its serum levels in all patients of our cohort were compared with serum lactate dehydrogenase (LDH) values, also taken at initial diagnosis of metastatic disease. As the statistical analysis shows, serum levels of ECP are independent of serum lactate dehydrogenase (Fig. 3).

Coefficient of determination r2 was 0.026, meaning only 2.6% of the variation on ECP levels can be explained by variations of LDH levels.

## Claims

1. A method of assessing the status of a malignant tumor, preferably melanoma, of a patient, the method comprising determining the level of expression or concentration of eosinophil activation proteins, preferably of eosinophil cationic protein (ECP), *in vitro* in a sample of the patient.

2. Eosinophil activation proteins, preferably eosinophil cationic protein (ECP), for use as a biomarker in a method of assessing the status of a malignant tumor, preferably melanoma, wherein the use comprises determining the level of expression or concentration of eosinophil activation proteins, preferably of eosinophil cationic protein (ECP), wherein the determination is practised on the human or animal body.

3. The method of claim 1 or the eosinophil activation proteins for use according to claim 2, wherein the method of assessing the status of a malignant tumor, preferably melanoma, is a method of prognosis of survival of a patient diagnosed with a malignant tumor, preferably melanoma, or alternatively a method of predicting response to therapy of a patient diagnosed with a malignant tumor, preferably melanoma, or alternatively a method of classifying a stage, preferably a prognostic stage, of a patient diagnosed with a malignant tumor, preferably melanoma, or alternatively a method of selecting the mode of treatment of a patient diagnosed with a malignant tumor, preferably melanoma, or alternatively a method of monitoring disease control in tumor patients, preferably melanoma patients.

4. The method of any of claims 1 or 3, or the eosinophil activation proteins for use according to any of claims 2 or 3, wherein the level of expression or concentration of eosinophil activation proteins, preferably of eosinophil cationic protein (ECP), is associated with survival or correlated to response to therapy.

5. The method of any of claims 1, 3 or 4, or the eosinophil activation proteins for use according to any of claims 2 to 4, wherein the level of the eosinophil activation proteins or mRNA coding for eosinophil activation proteins, preferably of eosinophil cationic protein (ECP) or mRNA coding for eosinophil cationic protein (ECP) is determined in a tumor tissue sample or in a body fluid, preferably in blood, serum, urine, or plasma of a patient, more preferably in the serum of a patient.

6. The method of any of claims 1 or 3 to 5, or the eosinophil activation proteins for use according to any of claims 2 to 5, wherein the level of eosinophil activation proteins, preferably eosinophil cationic protein (ECP), associated with a negative status, preferably a negative prognosis of survival or a negative prediction for response to therapy, is above 10 ng/ml, 16 ng/ml, 20 ng/ml, 25 ng/ml, 40 ng/ml, or 60 ng/ml, preferably above 16 ng/ml or 10 ng/ml, more preferably above 10 ng/ml.

7. The method of any of claims 1 or 3 to 6, or the eosinophil activation proteins for use according to any of claims 2 to 6, wherein the level of expression or concentration of eosinophil activation proteins is determined by a method indicative of expression of eosinophil activation proteins in the patient, preferably wherein the level of expression or concentration of eosinophil activation proteins is determined by RNA analysis, more preferably wherein the eosinophil activation protein is eosinophil cationic protein (ECP).

8. The method of any of claims 1 or 3 to 7, or the eosinophil activation proteins for use according to any of claims 2 to 7, wherein the level of eosinophil activation proteins, preferably eosinophil cationic protein (ECP), associated with a positive status, preferably a positive prognosis of survival or a positive prediction for response to therapy, is below 60 ng/ml, 40 ng/ml, 25 ng/ml, 20 ng/ml, 16 ng/ml, or 10 ng/ml, preferably below 16 ng/ml or 10 ng/ml, more preferably below 10 ng/ml.

9. The method of any of claims 1 or 3 to 9, or the eosinophil activation proteins for use according to any of claims 2 to 9, wherein the patient status is determined in comparison to the general comparative patient population at the time of diagnosis of disease, preferably wherein a positive and/or a negative status is determined in comparison to the general comparative patient population at the time of diagnosis of disease, more preferably wherein the time of diagnosis of metastatic disease is defined as within 0-6 months from the date of diagnosis of disease or before start of a treatment or during a treatment, particularly preferably within 0-6 months from the date of diagnosis of stage IV melanoma.

10. The method of any of claims 1 or 3 to 9, or the eosinophil activation proteins for use according to any of claims 2 to 9, wherein the level of expression or concentration of eosinophil activation proteins, preferably eosinophil cationic protein (ECP), is determined by using a qualitative or semiquantitative or quantitative method selected from fluoroenzyme immunoassay, immunohistochemistry, Western blot, flow cytometry, cytokine bead array analysis or ELISA, preferably wherein the level of expression or concentration of eosinophil activation proteins, preferably eosinophil cationic protein (ECP), is determined by using ELISA.

11. A kit for *in vitro* determination of the level of eosinophil activation proteins or mRNA of eosinophil activation proteins, preferably of cationic protein (ECP) or ECP mRNA, in a sample of a cancer patient.

12. The kit of claim 11, wherein the kit comprises reagents comprising one or more recognition molecules specifically binding to eosinophil activation proteins, preferably eosinophil cationic protein (ECP), wherein the reagents are suitable to detect eosinophil activation proteins, preferably eosinophil cationic protein (ECP) in a patient sample, preferably wherein the reagents are suitable for quantification of the level of eosinophil activation proteins, preferably eosinophil cationic protein (ECP), in a patient sample.

13. The kit of claim 11 or 12, wherein the patient is suffering from a malignant tumor, preferably melanoma, more preferably wherein the patient has been diagnosed with metastatic disease, even more preferably wherein the patient has been diagnosed within 0-6 months or before start of a treatment or during a treatment.

14. The kit of any of claims 11 to 13, wherein the patient sample is tumor tissue or a body fluid, preferably blood, serum or plasma of a patient, more preferably serum of a patient.

15. Use of the kit ofany of claims 11 to 14 for assessing the status of a malignant tumor, preferably for prognosis of survival of a patient diagnosed with a malignant tumor, preferably melanoma, and/or for predicting response to therapy of a patient diagnosed with a malignant tumor, preferably melanoma, and/or for classifying a stage of a patient diagnosed with a malignant tumor, preferably melanoma, and/or for selecting the mode of treatment of a patient diagnosed with a malignant tumor, preferably melanoma, or for monitoring disease control of a patient diagnosed with a malignant tumor, preferably melanoma.
